# EUROPEAN PATENT APPLICATION

(11) **EP 1 522 280 A2**
(43) Date of publication of application: **13.04.2005**
(21) Application number: 04021575.8
(22) Date of filing: 10.09.2004
(51) Int. Cl.: A61F 2/30

(54) **Medial/distal tip for artificial joint**

(30) Priority: 12.09.2003 JP 2003321002
(71) Applicant: Katsuya, Toshinobu, Kobe-shi, Hyogo 658-0073 (JP)
(72) Inventor: Katsuya, Toshinobu, Kobe-shi, Hyogo 658-0073 (JP)
(74) Representative: Reinhard - Skuhra - Weise & Partner GbR

(57) **Abstract**

The present invention provides a medial/distal tip for artificial joint that is capable of reliably guiding the insertion of the stem of a cementless artificial joint into a bone canal and holding the inserted stem at a stable position, thereby to prevent osteolysis and loosening of the artificial join from occurring and cause the bone to restore in thinned portion, chipper portion and/or weakened portion thereof by means of the medial/distal tip.

The medial/distal tip 10 for artificial joint is mounted in medial portion and/or the distal portion of the stem 2 of the cementless artificial joint thereby to guide the stem 2 of the artificial joint so that the distal end portion 2a of the stem 2 does not to make direct contact with the inner surface of the bone canal 4 of the bone when the stem 2 of the artificial joint is inserted into the bone canal 4, and make stable positioning of the stem 2 in the bone canal 4 when the insertion of the stem is completed, wherein the medial/distal tip 10 is made of a bone substituting material. The medial/distal tip 10 is also made in two-layer structure having an outer layer made of a bone substituting material and an inner layer made of a biodegradable and absorbable material.

## Description

### BACKGROUND OF THE INVENTION

### 1. Field of the Invention

The present invention relates to a medial/distal tip for artificial joint and, more particularly, to a medial/distal tip that can be mounted on a medial portion or a distal portion of a stem of a cementless artificial joint.

### 2. Description of Related Art

Artificial joints can be roughly divided into cemented artificial joint which is inserted into a bone canal that is filled with cement so that clearance between the bone and the stem of the artificial joint inserted therein is filled and fixed with the cement, and artificial joint of cementless type that is designed for directly joining the stem of the artificial joint with the bone without using cement.

In the case of artificial joint of cementless type, stable connection of the artificial joint and the bone is achieved by the cancellous bone joining with the proximate portion of the stem, although the cancellous bone takes several months to grow at the earliest.

When an artificial joint of cementless type is applied to the hip joint, for example, a stem which is made thicker not only in the proximate portion but also in the distal portion has been used for femoral bone, in order to increase the occupancy ratio of the artificial joint stem in the bone canal of the femoral bone stem. Since the bone canal has anatomical curve, however, distal end of the artificial hip joint stem comes into contact with the bone inside the bone canal to cause concentration of stress at the contact point, resulting in osteolysis which leads to loosening of the artificial joint, pain and/or bone fracture.

In order to overcome the problem described above, an artificial joint having a stem that tapers off in the distal portion has been provided. However, the stem which becomes thinner toward the distal end makes it difficult to precisely locate the narrower distal end portion of the stem at the center of the bone canal when inserting the stem into the bone canal, and the stem angle tends to take a varus position, valgus position, anteversion position or retroversion position in the bone canal. This situation also leads to osteolysis and loosening of the artificial joint.

In order to solve this problem, such a method has been provided as a metallic distal tip called the centralizer is mounted on the stem near the distal end thereof in a cementless artificial joint, and thereby to position the distal end of the stem is located at the center of bone canal. With this method, however, the distal tip mounted on the stem comes into contact with the bone thus resulting in the concentration of stress at the contact point, again causing osteolysis and loosening of the artificial joint.

With such a background as described above, it requires expedient to implant an artificial hip joint of cementless type, and stems without distal tips are used in most of implanting surgical operations at present.

Artificial joints of cemented type, on the other hand, is used in such a way as the bone canal is filled with cement so as to fix the stem inserted therein and the bone with each other.

For the artificial joints of cemented type, too, such a stem is provided that has a distal tip, made of a plastic or cement of the same kind as the filler cement, is mounted as a centralzer near the distal end of the stem. Role of the distal tip in the artificial joint of cemented type is mainly to position the distal end of the stem at the center of the bone canal, thereby to evenly distribute the filler cement around it. In the artificial joints of cemented type, the inserted stem and the cement are integrated into a single block.

Japanese Unexamined Patent Publication (Kokai) No. 10-309297 discloses an invention related to an artificial joint of cemented type, wherein a plug made of a material which can be decomposed and absorbed in living tissue is inserted in the bone canal in advance, so that cement does not enter too deep in the bone canal when the bone canal is filled with the cement. However, the plug made of a biodegradable and absorbable material is an exclusive attachment for the artificial joints of cemented type, and is used for the purpose of preventing the cement from falling off.

In the meantime, a variety of materials have been provided as the bone supplementing material which is also used according to the present invention.
[Patent Document 1] Japanese Unexamined Patent Publication (Kokai) No. 8-300126
[Patent Document 2] Japanese Unexamined Patent Publication (Kokai) No. 9-220656
[Patent Document 3] Japanese Unexamined Patent Publication (Kokai) No. 2003-073768
[Patent Document 4] Japanese Unexamined Patent Publication (Kokai) No. 10-314295
[Patent Document 5] Japanese Unexamined Patent Publication (Kokai) No. 2001-259016
[Patent Document 6] Japanese Unexamined Patent Publication (Kokai) No. 2001-46489
[Patent Document 7] Japanese Unexamined Patent Publication (Kokai) No. 2003-38636
[Patent Document 8] Japanese Unexamined Patent Publication (Kokai) No. 2003-14644
[Patent Document 9] Japanese Unexamined Patent Publication (Kokai) No. 2003-210568

With the artificial joint of cementless type, as described above, although it is made easier to position the distal end portion of the stem at the center of the bone canal by mounting the distal tip on the stem of the artificial joint, there has been such a problem that the distal tip makes contact with the bone over a long period of time so as to cause concentration of stress, thus resulting in osteolysis and loosening of the artificial joint.

In the case of the artificial joint of cemented type, on the other hand, since the stem is integrated with the filler cement, concentration of stress due to the use of the distal tip and the accompanying troubles such as osteolysis and loosening of the artificial joint do not pose significant problems from the first.

### BRIEF SUMMARY OF THE INVENTION

Accordingly, an object of the present invention is to eliminate the drawbacks and problems of the conventional cementless artificial joint described above and provide a medial/distal tip for artificial joint that is capable of reliably guiding the insertion of the stem of the cementless artificial joint and holding the stem that is inserted into the bone canal at an ideal and stable position, and enables the bone to restore in thinned, chipped and/or weakened potions thereof without allowing osteolysis and loosening of the artificial join that have been taking place in the prior art to occur even when the tip is mounted on the medial portion and/or the distal portion of the stem.

### BRIEF DESCRIPTION OF THE DRAWINGS

Fig. 1 is a sectional view showing the artificial joint stem having the medial/distal tip of the present invention mounted thereon inserted in the bone canal of femoral bone.
Fig. 2 shows two basic embodiments of the medial/distal tip in perspective view, Fig. 2 (A) being a perspective view of the medial/distal tip made solely of a bone substituting material and Fig. 2(B) being a perspective view of the medial/distal tip having two-layer structure made of a bone substituting material and a biodegradable and absorbable material.
Figs. 3(A) to (F) are perspective views showing embodiments of the medial/distal tip employing fitting hole.
Figs. 4 (A) and (B) are perspective views showing embodiments of the medial/distal tip of screw type.
Fig. 5 is a plan view showing an embodiment wherein a fitting hole of the medial/distal tip is offset from the center axis.
Fig. 6 shows another embodiment wherein a fitting hole of the medial/distal tip is offset from the center axis thereof, Fig. 6 (A) being a plan view of the medial/distal tip and Fig. 6(B) being a longitudinal sectional view thereof.
Fig. 7 shows further another embodiment wherein a fitting hole of the medial/distal tip is offset from the center axis thereof, Fig. 7 (A) being a plan view of the medial/distal tip and Fig. 7(B) being a longitudinal sectional view thereof.
Fig. 8 shows still another embodiment wherein a fitting hole of the medial/distal tip is offset from the center axis thereof, Fig. 8(A) being a plan view of the medial/distal tip and Fig. 8(B) being a longitudinal sectional view thereof.
Fig. 9 is a plan view showing an embodiment wherein a ridge protruding toward the center axis is formed on the inner circumference of the fitting hole of the medial/distal tip.
Fig. 10 is a plan view showing an embodiment wherein outer circumferential surface of the medial/distal tip is formed in an uneven shape.
Fig. 11 shows another embodiment of the medial/distal tip, Fig. 11(A) being a plan view and Fig. 11(B) being a horizontal sectional view.

### Description of Reference Numerals

- 1:: Epiphysis dome
- 2:: Stem
- 2a:: Distal end portion
- 2b:: Fitting hole
- 2c:: Fitting projection
- 3:: Thighbone
- 3a:: Weakened portion
- 4:: Bone canal
- 5:: Porous surface
- 10:: Medial/distal tip
- 10a:: Outer layer
- 10b:: Inner layer
- 11:: Fitting hole
- 12:: Male screw
- 13:: Female screw
- 14:: Fitting projection
- 15:: Minor fitting hole
- 16:: Ridge
- 17:: Bulge

- A:: Proximate portion
- B:: Medial/distal portion
- P:: Center axis
- Q:: Off-centered position
- L1:: Inner distance
- L2:: Outer distance
- L3:: Right-hand distance
- L4:: Left-hand distance

### DETAILED DESCRIPTION OF THE PREFRRED EMBODIMENTS

In order to achieve the object described above, first feature of the present invention is a medial/distal tip for artificial joint, that is mounted on the medial portion and/or the distal portion of the stem of a cementless artificial joint thereby to guide the distal end portion of the stem so as not to make contact with the inner surface of the bone canal (thus causing damage thereto 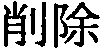 ) when the stem of the artificial joint is inserted into the bone canal, and make ideal and stable positioning of the stem in the bone canal when insertion of the stem is completed, wherein the medial/distal tip is made of a bone substituting material.

Second feature of the present invention is a medial/distal tip for artificial joint, that is mounted on the medial portion and/or the distal portion of the stem of a cementless artificial joint thereby to guide the distal end portion of the stem so as not to make direct contact with the inner surface of the bone canal (thus causing damage thereto 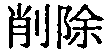 ) when the stem of the artificial joint is inserted into the bone canal, and make stable positioning of the stem in the bone canal when insertion of the stem is completed, wherein the medial/distal tip has two-layer structure of which outer layer is made of a bone substituting material and inner layer is made of a biodegradable and absorbable material.

Third feature of the present invention is a medial/distal tip for artificial joint wherein, in addition to the first or second feature described above, the bone substituting material is a bioabsorbable bone substituting material.

Fourth feature of the present invention is a medial/distal tip for artificial joint wherein, in addition to any one of the first to third features described above, the bone substituting material is a porous structured bone substituting material.

Fifth feature of the present invention is a medial/distal tip for artificial joint wherein, in addition to any one of the first to fourth features described above, the medial/distal tip is made in such a configuration as the outer diameter thereof at the mounting position is larger than the outer diameter of the stem, and the medial/distal tip is provided with mounting means comprising a fitting hole, a fitting projection, a screw or the like that engages with the stem.

Sixth feature of the present invention is a medial/distal tip for artificial joint wherein, in addition to the fifth feature described above, the mounting means for the medial/distal tip comprises a fitting hole, a fitting projection or a screw that is mounted on the medial/distal tip at a position offset from the center axis of the medial/distal tip.

Seventh feature of the present invention is a medial/distal tip for artificial joint wherein, in addition to the fifth or sixth feature described above, the mounting means for the medial/distal tip comprises a fitting hole into which a projection or ridge that protrudes from the inner circumferential surface toward the center is fitted.

Eighth feature of the present invention is a medial/distal tip for artificial joint wherein, in addition to any one of the first to seventh features described above, outer circumference of the medial/distal tip is made in such a configuration that has a cross section of a polygonal or irregular shape other than circle.

Ninth feature of the distal tip for artificial joint of the present invention is, in addition to any one of the first to fourth features, that the medial/distal tip is made in pin or peg shape that is attached to the stem of the artificial joint on the lateral surface thereof in the medial or distal portion.

The medial/distal tip for artificial joint described in claim 1 is mounted on the medial portion and/or the distal portion of the stem of a cementless artificial joint and enables it to guide the distal end portion of the stem so as not to make direct contact with the inner surface of the bone canal and stably position the stem in the bone canal when the stem of the artificial joint is inserted into the bone canal, while the medial/distal tip is made of a bone substituting material so that, when the medial/distal tip is mounted on the stem of a cementless artificial joint, the distal end portion of the stem can be guided so as not to make direct contact with the inner surface of the bone canal as the artificial joint stem is inserted into the bone canal. It is also made possible to stably position the stem of the cementless artificial joint at a desirable balanced position in the bone canal.

In addition, since the medial/distal tip is made of a bone substituting material, it is made possible to eliminate the possibility of osteolysis in the cementless artificial joint, loosening of the artificial joint, pain and/or bone fracture due to the contact of the medial/distal tip with the bone.

Moreover, since the medial/distal tip for artificial joint stem is made of a bone substituting material, the portion of the bone where the medial/distal tip is located eventually integrates with the medial/distal tip on the inner surface of the bone and grows to become thicker, so that the bone restores and becomes stronger in thinned, chipped and/or weakened portions thereof.

The medial/distal tip for artificial joint described in claim 2 is mounted on the medial portion and/or the distal portion of the stem of a cementless artificial joint thereby to guide the medial and distal end portion of the stem so as not to make direct contact with the inner surface of the bone canal when the stem of the artificial joint is inserted into the bone canal, and make stable positioning of the stem in the bone canal when insertion of the stem is completed, wherein the medial/distal tip has two-layer structure of which outer layer is made of a bone substituting material and the inner layer is made of a biodegradable and absorbable material.

Therefore, when the medial/distal tip is mounted on the stem of a cementless artificial joint, it is made possible to guide the distal end portion of the stem so as not to make contact with the inner surface of the bone canal and stably position the stem of the cementless artificial joint stem at a desirable balanced position in the bone canal, similarly to the invention described in claim 1.

Furthermore, since the outer layer of the medial/distal tip for artificial joint stem is made of a bone substituting material, the portion of the bone where the medial/distal tip is located eventually integrates with the medial/distal tip on the inner surface of the bone and grows to become thicker, so that the bone restores and becomes stronger in thinned, chipped and/or weakened portions thereof.

Moreover, since the inner layer of the medial/distal tip is made of a biodegradable and absorbable material, the biodegradable and absorbable material that makes the inner layer of the medial/distal tip can be decomposed and absorbed in a period of several months after the surgery during which the proximate portion of the stem and the cancellous bone develop rigid fixation with each other or the bone substituting material bonds with the bone, thereby making it possible to eliminate the possibility of osteolysis in the cementless artificial joint, loosening of the artificial joint, pain and/or bone fracture due to the contact of the stem and the bone over a long period of time that is caused by separation of contact between the bone substituting material (bone) side and the stem.

According to the medial/distal tip for artificial joint described in claim 3, in addition to the effects of the invention of claim 1 or 2, since the bone substituting material is a bioabsorbable bone substituting material, the portion of the bone where the medial/distal tip is located is made thicker and stronger by the bone substituting material, and the bone substituting material is replaced by the real bone so that healthy bone is strengthened.

According to the medial/distal tip for artificial joint described in claim 4, in addition to the effects of any one of the invention of claim 1 to 3, since the bone substituting material is a porous structured bone substituting material, restoration of the bone is achieved more effectively by the porous structured material so that the bone is made thicker and stronger.

According to the medial/distal tip for artificial joint described in claim 5, in addition to the effects of any one of the invention of claim 1 to 4, since the medial/distal tip is made in such a configuration as the outer diameter thereof at the mounting position is larger than the outer diameter of the stem, and the medial/distal tip is provided with mounting means comprising a fitting hole, a fitting projection, a screw or the like that engages with the stem, it is made possible to guide the distal end portion of the stem while surely preventing the distal end portion of the stem from making direct contact with the inner surface of the bone canal when the step of the artificial joint is inserted into the bone canal.

Also because the medial/distal tip that is larger than the outer diameter of the stem at the mounting position thereof is restricted from changing the position by the surrounding inner circumference of the bone canal, stem position is also restricted accordingly. That is, position of the stem in the bone canal upon completion of insertion of the stem is established stably by the medial/distal tip.

The medial/distal tip can be mounted on the stem easily and reliably by using the mounting means comprising a fitting hole, a fitting projection, a screw or the like that is provided on the medial/distal tip.

According to the medial/distal tip for artificial joint described in claim 6, in addition to the effects described in claim 5, since the mounting means comprises a fitting hole, a fitting projection or a screw that is provided on the medial/distal tip at a position offset from the center axis thereof, the mounting means can be mounted easily and reliably at a position offset from the center axis of the medial/distal tip by using a fitting hole, a fitting projection or a screw as the mounting means for the medial/distal tip.

By setting the mounting means on the medial/distal tip at a position offset from the center axis thereof, the distal portion of the stem placed in the bone canal can be stably positioned at a position offset from the center of the bone canal.

Since the position of the medial and distal portion of the stem in the bone canal can be set at a position offset from the center of the bone canal, the inserted stem can be held at a balanced and optimum position in the bone canal in accordance to the anatomical curve of the bone that varies among individuals.

Since human bones are curved to an extent that is different from one individual to the other, there may be such a case that it is preferable to dispose the medial and distal portion of the stem in the bone canal at a position which is somewhat offset from the center rather than at the center of the bone canal, in order to keep the stem at a favorable position where the inserted stem is held in well-balanced position as a whole in the bone canal. Since such a condition of the bone can be known by conducting X-ray imaging, CT, MRI or the like in advance, stable positioning of the distal end portion of the stem can be achieved at a predetermined position which is offset from the center of the bone canal by determining the preferable direction and amount of offset distance of the medial and distal portion of the stem from the center of the bone canal beforehand, and accordingly fitting the medial/distal tip into the stem while adjusting the azimuthal position of the medial/distal tip in the horizontal plane.

According to the medial/distal tip for artificial joint described in claim 7, in addition to the effects of the invention described in claim 5 or 6, it is made possible to fit the medial/distal tip in the stem more firmly and securely than in a case in which the inner circumferential surface of the fitting hole is a smooth circular surface when fitting the medial/distal tip onto the stem, by making the mounting means of the medial/distal tip in the form of fitting hole and providing a projection or a ridge that protrudes from the inner circumferential surface toward the center of the fitting hole. In addition, forming the projection or ridge in the fitting hole ensures the flow of bone marrow after the fitting hole is mated with the stem.

According to the medial/distal tip for artificial joint described in claim 8, in addition to the effects of the invention described in any one of claims 1 to 7, it is made possible to ensure sufficient flow of bone marrow in the bone canal without obstruction, even when the medial and distal portion of the stem is positioned by the medial/distal tip in the bone canal, since the outer circumference of the medial/distal tip is formed in polygonal or other uneven shape other than circle.

According to the medial/distal tip for artificial joint described in claim 9, in addition to the effects of the invention described in any one of claims 1 to 4, since the medial/distal tip is made in pin or peg shape that is attached to the stem of the artificial joint on the lateral surface thereof in the medial and/or distal portion, the portion of the bone where the medial/distal tip in pin or peg shape is located grows to become thicker, so that the bone restores and becomes stronger in thinned, chipped and/or weakened portions thereof.

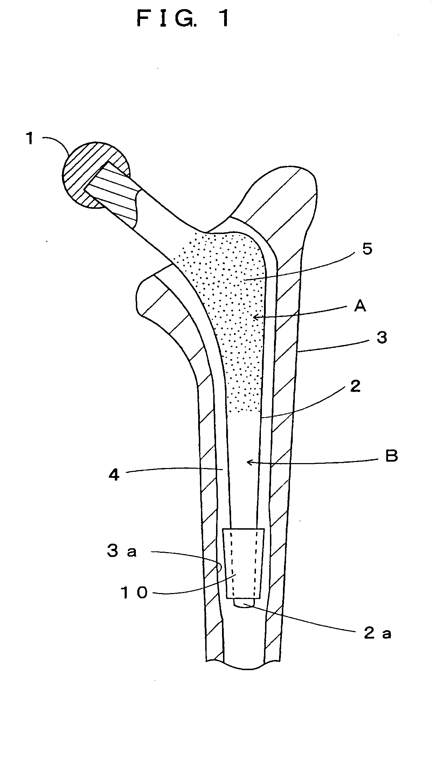

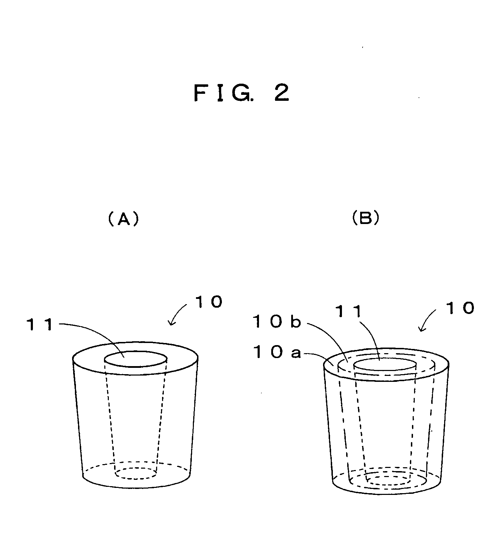

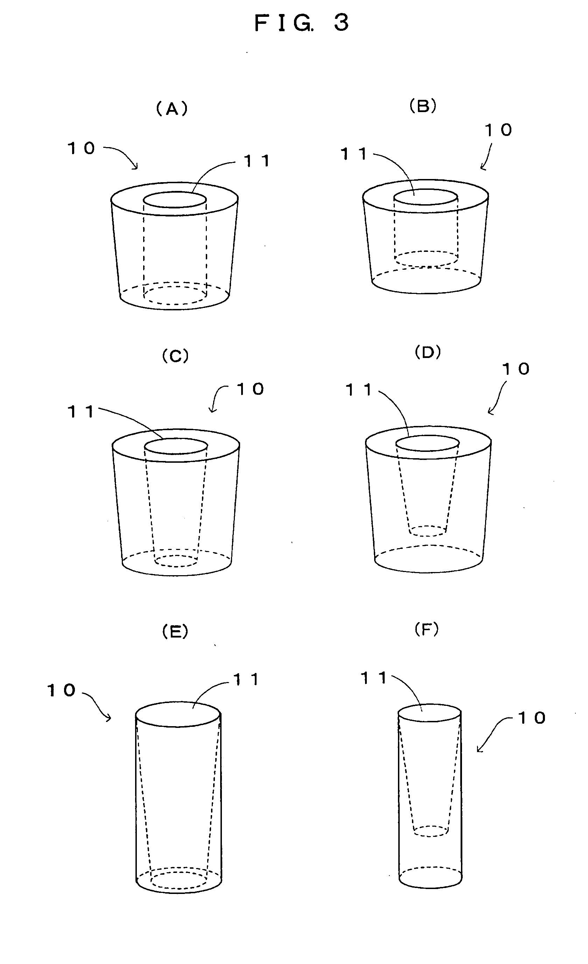

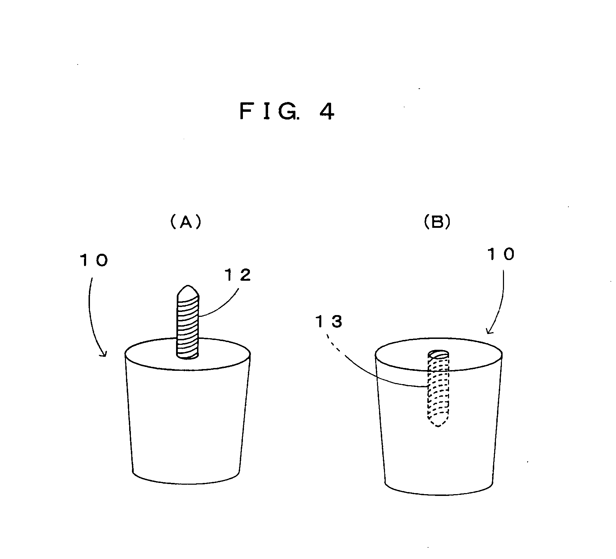

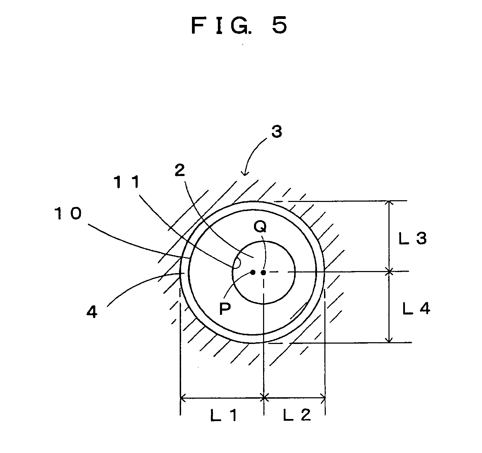

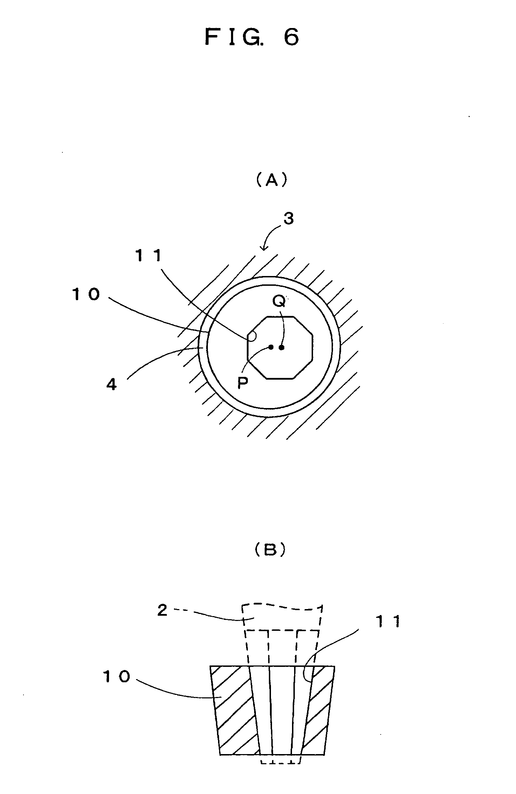

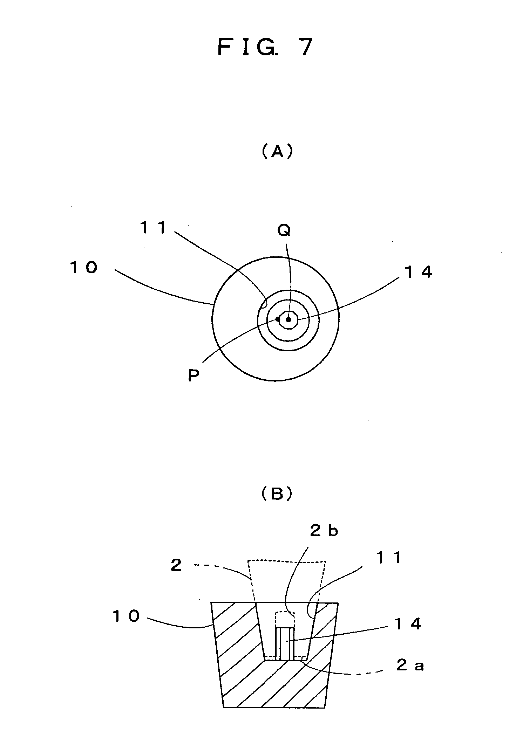

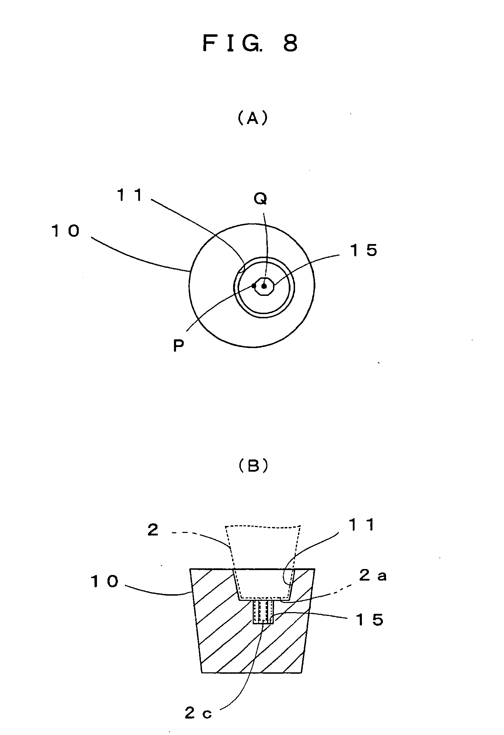

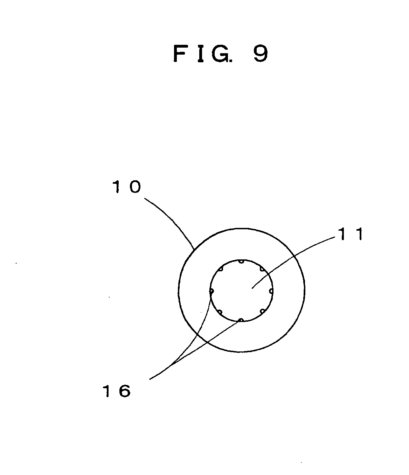

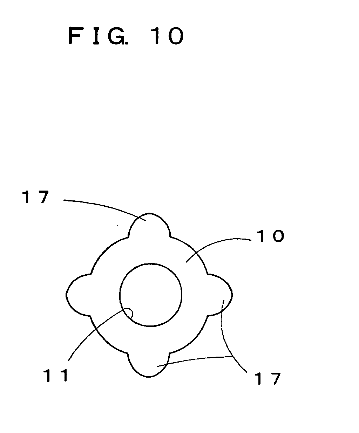

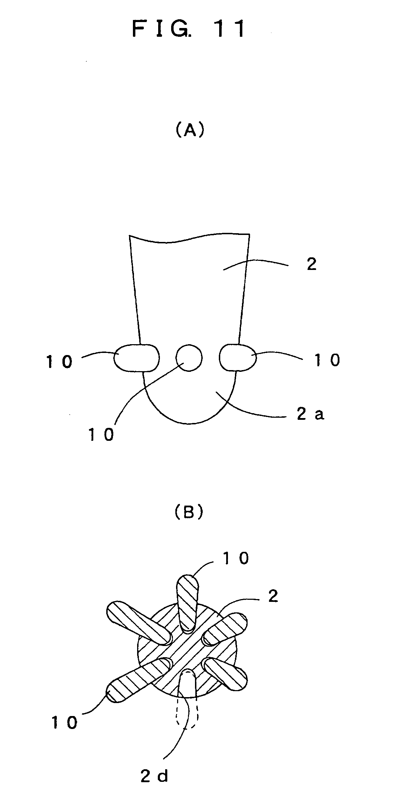

Fig. 1 is a sectional view showing the artificial joint stem having the medial/distal tip of the present invention mounted thereon being inserted in the bone canal of femoral bone. Fig. 2 shows two basic embodiments of the medial/distal tip in perspective view, Fig. 2 (A) being a perspective view of the medial/distal tip made solely of a bone substituting material, Fig. 2(B) being a perspective view of the medial/distal tip having two-layer structure made of a bone substituting material and a biodegradable and absorbable material. Figs. 3(A) to (F) are perspective views showing various embodiments of the medial/distal tip employing a fitting hole. Figs. 4 (A) and (B) are perspective views showing embodiments of the medial/distal tip of screw type. Fig. 5 is a plan view showing a medial/distal tip of an embodiment wherein a fitting hole of the medial/distal tip is offset from the center axis thereof. Fig. 6 shows another embodiment wherein a fitting hole of the medial/distal tip is offset from the center axis thereof, Fig. 6 (A) being a plan view of the medial/distal tip and Fig. 6(B) being a longitudinal sectional view thereof. Fig. 7 shows another embodiment wherein a fitting hole of the medial/distal tip is offset from the center axis thereof, Fig. 7(A) being a plan view of the medial/distal tip and Fig. 7(B) being a longitudinal sectional view thereof. Fig. 8 shows another embodiment wherein a fitting hole of the medial/distal tip is offset from the center axis thereof, Fig. 8(A) being a plan view of the medial/distal tip and Fig. 8(B) being a longitudinal sectional view thereof. Fig. 9 is a plan view showing an embodiment wherein a ridge protruding toward the center axis is formed on the inner circumferential surface of the fitting hole of the medial/distal tip. Fig. 10 is a plan view showing an embodiment wherein outer circumferential surface of the medial/distal tip is formed in an uneven shape. Fig. 11 shows another embodiment of the medial/distal tip, Fig. 11(A) being a plan view and Fig. 11(B) being a horizontal sectional view.

First, with reference made to Fig. 1, reference numeral 1 denotes a ball head of artificial joint, 2 denotes a stem of the artificial joint, 3 denotes a proximal part of femoral bone, 4 denotes a bone canal and 5 denotes circumferential surface processed to be porous in the proximal portion of the artificial hip joint stem 2.

A medial/distal tip 10 is mounted on the stem 2 of the artificial hip joint in a medial and/or distal portion B beyond a proximal portion A of which circumferential surface 5 is processed to be porous, specifically at a position near the distal end 2a thereof.

The artificial joint stem 2 having the medial/distal tip 10 mounted thereon is inserted into the bone canal 4 from the proximal part of femoral bone 3 during a surgical operation.

When the artificial joint stem 2 is inserted into the bone canal 4, the medial/distal tip 10 guides the insertion of the stem 2 while preventing the distal end portion 2a of the stem 2 from making direct contact with the inner surface of the bone canal of the femoral bone 3 thereby causing a damage and from obstructing smooth insertion of the stem 2.

Outer diameter of the medial/distal tip 10 is made larger than the outer diameter of the stem 2 at the mounting position thereof. More specifically, since the medial/distal tip 10 is made of a bone substituting material at least in its outer layer, as will be described later, profile of the medial/distal tip 10 preferably has such a diameter as the bone substituting material touches the inner surface of the bone canal 4 of the femoral bone 3.

When insertion of the artificial joint stem 2 into the bone canal 4 is completed, outer circumferential surface of the medial/distal tip 10 either touches the inner circumferential surface of the bone canal of the femoral bone 3 or faces therewith with a small clearance in-between, so that displacement of the medial/distal tip 10 is constrained within a small region, thereby achieving stable and balanced positioning of the distal end portion 2a of the stem 2, too.

The inner circumferential surface of the bone canal of the femoral bone 3 (inner surface of the femoral bone 3) may be reamed in advance, so as to just fit the medial/distal tip 10 mounted on the stem 2. This would bring the medial/distal tip 10 in contact with the inner surface of the bone when the stem 2 is inserted into the bone canal 4 of the femoral bone 3, so that the natural bone grows earlier.

The stem 2 of the artificial joint may be made of a metallic material such as titanium alloy, cobalt-chromium alloy or stainless steel, but may also be made of other metallic material or non-metallic material having high strength.

Referring to Fig. 2, the medial/distal tip 10 can be made solely of a bone substituting material as shown in (A). Alternatively, the medial/distal tip 10 may be formed in two-layer structure with an outer layer 10a made of a bone substituting material and an inner layer 10b made of a biodegradable and absorbable material as shown in (B). Reference numeral 11 denotes a fitting hole.

By using the bone substituting material in the medial/distal tip 10 which is positioned in a weakened portion 3a of the bone 3 where it is thinned, chipped or otherwise weakened, weakened portion 3a of the bone can be caused to grow to become thicker and stronger.

The bone substituting material may be ceramics such as alumina, zirconia or hydroxyapatite, plastics such as polymethyl methacrylate or high-density polyethylene, or other bone substituting material that has been developed or is expected to be developed in the future.

The bone substituting material may also be a bioabsorbable bone substituting material. For the bioabsorbable bone substituting material, a-tricalcium phosphate, hydroxyapatite, A-W glass ceramics or bioabsorbable bone substituting material that has been developed or is expected to be developed in the future.

The bone substituting material may also be a porous structured bone substituting material. The porous structured bone substituting material may be made by sintering a powdery or granular material such as a-tricalcium phosphate or hydroxyapatite.

The biodegradable and absorbable material used for the inner layer 10b may be PLLA (poly-L-lactic acid), lactic acid-glycolic acid copolymer or lactic acid-caprolactone copolymer, which may be used individually or in combination of two or more thereof. It needs not to say that other biodegradable and absorbable material that is known or would be developed in the future may also be used.

The biodegradable and absorbable material is hydrolyzed and absorbed in the bone canal 4 over time. Duration in which the inner layer 10b of the medial/distal tip 10 performs its function can be controlled by adjusting the proportion of the biodegradable and absorbable material, thereby controlling the rate of decomposition and absorption thereof in the living tissue. The duration in which the inner layer 10b of the medial/distal tip 10 performs its function refers to the period during which the cancellous bone grows onto the porous finished surface 5 of the proximate portion of the stem 2 and firmly joins therewith, so that the stem 2 and the femoral bone 3 develop rigid fixation with each other, and bone grows between the outer layer 10a and inner surface of the femoral bone canal. This period is 3 to 6 months in average while it depends on the individual.

Now making reference to Fig. 3, configuration of the medial/distal tip 10 that is made either solely of the bone substituting material or in two-layer structure of the bone substituting material and the biodegradable and absorbable material is inverted conical shape as shown in Fig. 3(A), having a fitting hole 11 formed to penetrate therethrough along the center axis. In the case of the medial/distal tip 10 shown in Fig. 3(A), the medial/distal tip 10 is mounted in the distal portion, in the medial portion or in a region that spans between the medial portion and the distal portion of the stem 2 while passing through the distal end portion 2a of the stem 2, for example, as shown in Fig. 1. In this case, the stem 2 is properly tapered from the distal end portion 2a to the medial and distal portion B thereof, so as to fix the medial/distal tip 10 thereby.

Configuration of the medial/distal tip 10 may be truncated inverted conical shape as shown in Fig. 3(B), having a fitting hole 11 that is formed along the center axis so as to stop midway without penetrating therethrough. The medial/distal tip 10 of this configuration can be mounted on the stem 2 in the distal portion or in a region from the distal portion to the medial portion thereof so as to cover the distal end portion 2a of the stem 2 from below.

Configuration of the medial/distal tip 10 may be truncated inverted conical shape as shown in Fig. 3(C), having a fitting hole 11 that is tapered off being formed to penetrate therethrough along the center axis. The medial/distal tip 10 of this configuration can be satisfactorily mounted on the stem 2 which is not tapered near the distal end portion 2a thereof.

Configuration of the medial/distal tip 10 may be truncated inverted conical shape as shown in Fig. 3(D), having a fitting hole 11 that is tapered off being formed along the center axis so as to stop at a mid point thereof without penetrating therethrough. The medial/distal tip 10 of this configuration can be mounted on the stem 2 in the distal portion or in a region from the distal portion to the medial portion thereof so as to cover the distal end portion 2a of the stem 2 from below.

In Figs. 3(A) to (D), the truncated inverted conical shape may be replaced by cylindrical shape, as a matter of course. Profile of the medial/distal tip 10 may have various shapes.

The medial/distal tip 10 has such a configuration as shown in Fig. 3(E), (F) where the top end of the fitting hole 11 of the medial/distal tip 10 has inner diameter near equal to the outer diameter of the top end of the medial/distal tip 10. In the cases shown in Figs. 3(E) and (F), too, the medial/distal tip 10 may be made in two-layer structure instead of being made solely of the bone substituting material. In this case, for example, the medial/distal tip 10 may be constituted from inner and outer layers, except for the upper portion thereof, with the thinner upper potion being made of the bone substituting material only. The configuration as shown in Fig. 3(E) or (F) makes it possible to connect the stem 2 and the distal tip 10 with substantially continuous surface without step at the joint.

The medial/distal tip 10, when made longer, can be mounted so as to cover a region that ranges over the medial and distal portion B of the stem 2. When the medial/distal tip 10 is made relatively shorter, it can be mounted either in the medial portion or in the distal portion of the stem 2.

Now referring to Fig. 4, the medial/distal tip 10 may be mounted on the stem 2 in the distal portion thereof also by means of a screw. Fig. 4(A) shows a male screw 12 provided at the top end of the medial/distal tip 10. With a female screw not shown in the drawing provided at the distal end portion 2a of the stem 2, the medial/distal tip 10 is mounted by screwing therein. Fig. 4(B) shows a female screw 13 provided at the top end of the medial/distal tip 10. With a male screw not shown in the drawing provided at the distal end portion 2a of stem 2, the medial/distal tip 10 is mounted by screwing both parts.

In case the medial/distal tip 10 is made in two-layer structure with the inner layer formed from the biodegradable and absorbable material, the screwed portion may be formed from the same bioabsorbable material. In case the medial/distal tip 10 is made of the bone substituting material only, the screwed portion may be formed from either the same material or a different material.

In the case of the medial/distal tip 10 shown in Fig. 3 and Fig. 4, the fitting hole 11, the male screw 12 or the female screw 13 is provided along the center axis of the medial/distal tip 10, although the fitting hole 11, the male screw 12 or the female screw 13 may also be provided at a position offset from the center axis of the medial/distal tip 10.

Fig. 5 shows an embodiment in which the fitting hole 11 of the medial/distal tip 10 is provided at a position Q offset from the center axis P of the medial/distal tip 10. Offsetting the fitting hole 11 of the medial/distal tip 10 from the center axis P of the medial/distal tip 10 enables it to freely adjust the position of the stem 2, that is inserted into the fitting hole 11, in the bone canal 4.

In other words, in the medial and distal portion B of the stem 2 wherein the medial/distal tip 10 is fitted, when the distance between the stem 2 and the inner circumferential surface of the femoral bone 3 is represented by inner distance L1, outer distance L2, right-hand distance L3 and left-hand distance L4, the distances L1, L2, L3 and L4 can be freely altered and adjusted. The alteration and adjustment can be made by turning the medial/distal tip 10 by an angle within 360 degrees in the horizontal plane when fitting the medial/distal tip 10 on the stem 2.

As will be seen from the description above, position of the medial and distal portion B of the stem 2 in the bone canal 4 can be freely adjusted so as to achieve positioning by using the medial/distal tip 10 that has the fitting hole 11 located at an offset position. This capability becomes very important when inserting the stem 2 into the bone canal 4 and positioning it with good balance as a whole in accordance to the anatomical curve of the individual human bone. Moreover, the stem 2 can be disposed in the bone canal 4 with good balance as a whole in conjunction with the adjustment of the stem 2 position in the proximate portion A (which can be done relatively easily because of visibility), when position of the medial and distal portion B of stem 2 in the bone canal 4 (position in the horizontal plane in the bone canal 4) can be adjusted to a favorable position. In addition, even when a weakened portion 3a of the femoral bone 3 is located unevenly along the circumferential direction in a portion of the femoral bone 3, the bone substituting material can be favorably positioned in the weakened portion 3a.

In the embodiment shown in Fig. 5, position of the mounting means in the form of fitting hole 11 is offset from the center axis P of the medial/distal tip 10, although the fitting hole 11 may be replaced with a screw such as the male screw 12 or the female screw 13 of the medial/distal tip 10 provided as the mounting means at a position offset from the center axis P.

The fitting hole 11 may also be replaced with a fitting protrusion provided as the mounting means at a position offset from the center axis P.

Fig. 6 shows an embodiment wherein the fitting hole 11 of the medial/distal tip 10 is provided at a position Q that is offset from the center axis P of the medial/distal tip 10, similarly to the embodiment shown in Fig. 5, except for making the shape of the fitting hole 11, specifically the horizontal section thereof, in a polygonal shape.

By employing the fitting hole 11 having polygonal shape, it is made possible to easily and surely fit and fix the medial/distal tip with respect to the medial and distal end portion of the stem 2 having the same shape of horizontal section. Particularly, the extent of projection of the medial/distal tip 10 around the stem 2 can be adjusted easily and reliably by turning the medial/distal tip 10 in a step of the angle of one apex of the polygon.

Although the fitting hole 11 of the distal tip 10 is formed in the tapered shape in this embodiment, the fitting hole may not necessarily be tapered but may be a straight hole.

In the embodiment shown in Fig. 7, the off-centered fitting hole 11 of the medial/distal tip 10 is formed with a bottom without penetrating therethrough, and a fitting projection 14 is provided so as to protrude from the bottom of the fitting hole 11, while the fitting projection 14 is inserted into the fitting hole 2b formed in the distal end portion 2a of the stem 2 thereby to mount the medial/distal tip 10 on the stem 2.

The fitting projection 14 is provided at a position Q offset from the center axis P of the medial/distal tip 10. Position of the distal end portion 2a of the stem 2 can be adjusted in the bone canal 4 by fitting the fitting projection 14 into the fitting hole 2a of the stem 2 while turning it in the circumferential direction and adjusting the position.

The fitting projection 14 is also made in a polygonal prism shape. Advantage of making it in a polygonal shape is the same as that described previously.

In the embodiment shown in Fig. 8, the off-centered fitting hole 11 of the medial/distal tip 10 is formed with a bottom without penetrating therethrough, and a minor fitting hole 15 is provided at the bottom of the fitting hole 11, so that a fitting projection 2c formed on the distal end portion 2a of the stem 2 is inserted into the minor fitting hole 15 thereby to mount the medial/distal tip 10 on the stem 2.

The minor fitting hole 15 is provided at a position Q offset from the center axis P of the medial/distal tip 10. Position of the distal end portion 2a of the stem 2 can be adjusted in the bone canal 4 by fitting the minor fitting hole 15 and the fitting projection 2c of the stem 2 while turning with respect to each other in the circumferential direction and adjusting the position.

The minor fitting hole 15 is also made in a polygonal shape. Advantage of making it in a polygonal shape is the same as that described previously.

As shown in Fig. 9, the fitting hole 11 of the medial/distal tip 10 may be provided with a ridge 16 formed in the longitudinal direction to protrude from the inner circumferential surface thereof toward the center. A projection may also be provided instead of the ridge 16.

It is made possible to mount the medial/distal tip 10 in the stem 2 more firmly and securely than in a case in which the inner circumferential surface of the fitting hole 11 is a smooth surface, by making the ridge 16 or projection that protrudes from the inner circumferential surface toward the center of the fitting hole 11 of the medial/distal tip 10.

As shown in Fig. 10, the outer circumference of the medial/distal tip 10 may not necessarily be circular. The outer circumference of the medial/distal tip 10 may be formed in polygonal or other uneven shape. In Fig. 10, bulges 17 are provided at equal intervals on the outer circumference of the medial/distal tip 10. By forming the outer circumference of the medial/distal tip 10 in uneven shape, it is made possible to allow bone marrow or the like to flow freely through the clearance among the unevenness. The uneven surface also enables it to position the medial/distal tip 10 so that the projections on the circumference of the medial/distal tip 10 come nearer to the partially weakened portion 3a of the bone.

Fig. 11 shows another embodiment of the medial/distal tip of the present invention. In this embodiment, the medial/distal tip 10 is made in pin shape. A plurality of the pin-shaped medial/distal tips 10 are prepared and are inserted into mounting holes 2d disposed at appropriate intervals (60-degree intervals in the case of this embodiment) along the circumference of the stem 2 near the distal portion (or medial portion) thereof for mounting. With the plurality of pin-shaped medial/distal tips 10 mounted in radial configuration on the circumference of the stem 2 in the distal portion or the medial portion thereof, the medial and distal portion B of the stem 2 can be located at a favorable position in stable condition in the bone canal 4.

Base-side end of the pin-shaped medial/distal tip 10 may also be threaded so as to be mounted by screwing into the threaded mounting hole 2d of the stem 2.

The pin-shaped medial/distal tips 10 may also be provided in plurality having different lengths, so that projecting lengths of the pin-shaped medial/distal tips 10 from the stem 2 are different along the circumference when the medial/distal tips 10 of different lengths are mounted in the mounting holes 2d of the stem 2. This constitution enables it to make stable positioning of the medial portion and the distal portion of the stem 2 at an off-centered position in the bone canal 4. During the positioning process, the off-centered position of the stem 2 in the bone canal 4 can be freely adjusted by selecting the position along the circumference of the stem 2 where one of the pin-shaped medial/distal tips 10 of a particular length is to be mounted.

The pin-shaped medial/distal tips 10 may not necessarily be mounted in radial configuration along the circumference at predetermined positions in the longitudinal direction of the stem 2 as shown in Fig. 11, since the pin-shaped medial/distal tip 10 can be located at any position where it is desired to substitute the bone. One or more medial/distal tip may be mounted at any position on the lateral surface of the stem 2 in the medial and distal portion B thereof, corresponding to the position where it is desired to substitute the bone.

The pin-shaped medial/distal tip 10 may of course be made solely of the bone substituting material, but it may also be made of the bone substituting material and the biodegradable and absorbable material. In the latter case, the outer layer of the medial/distal tip 10 near the femoral bone 3 is made of the bone substituting material and the inner layer of the medial/distal tip 10 near the stem 2 is made of the biodegradable and absorbable material.

While the pin-shaped medial/distal tip 10 can be applied locally to a position where it is desired to substitute the femoral bone 3, peg shaped medial/distal tip 10 may also be used to perform the function of the medial/distal tip 10 applied to a position where it is desired to substitute the bone 3. The peg shaped medial/distal tip 10 may be mounted on the lateral surface of the stem 2 that corresponds to the portion of the femoral bone 3 where it is desired to substitute, and the bone can be substituted in the portion.

The medial/distal tip 10 of the present invention may be applied, besides the artificial hip joint, to artificial shoulder joint, artificial elbow joint, artificial wrist joint, artificial finger joint, artificial knee joint and artificial ankle joint.

## Claims

1. A medial/distal tip for artificial joint that is mounted on a cementless artificial joint in the medial portion and/or the distal portion of the stem thereof and guides the distal end portion of the stem so as not to make direct contact with the inner surface of a bone canal when said artificial joint stem is inserted into the bone canal and, at the same time, achieves stable positioning of the stem in the bone canal when the insertion of the step is completed, wherein said medial/distal tip is made of a bone supplementing material.

2. A medial/distal tip for artificial joint that is mounted on a cementless artificial joint in the medial portion and/or the distal portion of the stem thereof and guides the distal end portion of the stem so as not to make direct contact with the inner surface of a bone canal when said artificial joint stem is inserted into the bone canal and, at the same time, achieves stable positioning of the stem in the bone canal when the insertion of the step is completed, wherein said medial/distal tip is made in two-layer structure having an outer layer made of a bone substituting material and an inner layer made of a biodegradable and absorbable material

3. The medial/distal tip for artificial joint according to claim 1 or 2, wherein said bone substituting material is a bioabsorbable bone substituting material.

4. The medial/distal tip for artificial joint according to any one of claims 1 to 3, wherein said bone substituting material is a porous structured bone substituting material.

5. The medial/distal tip for artificial joint according to any one of claims 1 to 4, wherein the medial/distal tip is made in such a configuration as the outer diameter thereof is larger than the outer diameter of the stem at the mounting position, and the medial/distal tip has mounting means such as a fitting hole, a fitting protrusion, a screw or the like that fits with the stem.

6. The medial/distal tip for artificial joint according to claim 5, wherein the mounting means of the medial/distal tip is a fitting hole, a fitting protrusion or a screw provided on the medial/distal tip at a position that is offset from the center axis of the medial/distal tip.

7. The medial/distal tip for artificial joint according to claim 5 or 6, wherein the mounting means of the medial/distal tip is a fitting hole which has a projection or a ridge that protrudes from the inner circumferential surface toward the center of the fitting hole.

8. The medial/distal tip for artificial joint according to any one of claims 1 to 7, wherein the medial/distal tip is made in such a configuration as the outer circumference has polygonal or other uneven shape other than circle.

9. The medial/distal tip for artificial joint according to any one of claims 1 to 4, wherein the medial/distal tip is made in pin or peg shape and is designed for mounting on the lateral surface of medial/distal portion of artificial joint stem.
